(19) 

Europäisches
Patentamt
European
Patent Office
Office européen
des brevets

(11) **EP 1 655 308 B1**

(12) **EUROPEAN PATENT SPECIFICATION**

(45) Date of publication and mention
of the grant of the patent:
**15.07.2009 Bulletin 2009/29**

(51) Int Cl.:
*C07K 14/77* *(2006.01)*    *A61K 38/38* *(2006.01)*
*A23J 1/00* *(2006.01)*

(21) Application number: **05380108.0**

(22) Date of filing: **26.05.2005**

(54) **Adducts of soluble ovalbumin with trivalent iron polyalcohol complexes and method to produce them**

Addukte von löslichem Ovalbumin mit Eisen-Polyalkohol-Komplexen und Verfahren zu deren Herstellung

Produits d'addition d'ovalbumine soluble et de complexes polyalcool/fer trivalent et leur procédé de préparation

(84) Designated Contracting States:
**AT BE BG CH CY CZ DE DK EE ES FI FR GB GR HU IE IS IT LI LT LU MC NL PL PT RO SE SI SK TR**

(30) Priority: **03.11.2004 ES 200402640**

(43) Date of publication of application:
**10.05.2006 Bulletin 2006/19**

(73) Proprietors:
• **Tedec-Meiji Farma, S.A.**
  **28802 Alcalà de Henares (ES)**
• **Syntex Uruguaya, S.A.**
  **1100 Montevideo (UY)**

(72) Inventor: **Diaz, Victor Bautista**
**1100 Montevideo (UY)**

(74) Representative: **Gil-Vega, Victor**
**Corazón de Maria, 6**
**28002 Madrid (ES)**

(56) References cited:
**EP-A- 0 357 776        EP-A- 0 875 249**
**ES-A1- 2 123 432        ES-A1- 2 188 336**

• **JAE-SEUNG Y ET AL: "Changes in biochemical properties of ovomucoid by radiation" RADIATION PHYSICS AND CHEMISTRY, ELSEVIER SCIENCE PUBLISHERS BV., AMSTERDAM, NL, vol. 48, no. 6, December 1996 (1996-12), pages 731-735, XP004070747 ISSN: 0969-806X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**OBJECT OF THE INVENTION**

The present invention relates to a procedure to obtain adducts of soluble ovalbumin with complexes formed by polyalcohols derived from monosaccharides and trivalent iron, for their subsequent use in both pharmaceutical and dietetic compositions, which will be used in the therapeutic treatment of iron deficiency in persons or animals.

More specifically, the purpose of this invention is a method for the treatment of ovalbumin which allows for its solubilisation without greatly affecting its molecular weight, as well as eliminating allergic and immunotoxicity conditions associated with said ovalbumin, and the elimination, within the field of industrial preparation, of gelling conditions which make batches useless and of random appearance phenomena, due to the incontrollable presence of impurities naturally present in commercial ovalbumin.

**BACKGROUND OF THE INVENTION**

Albumin is a carrier, i.e. a physiological vehicle or transporter which, as is well known, internally carries products which are in themselves insoluble, such as fatty acids, steroids, hydroxylated compounds, etc. Thus, albumins in general perform functions of storage, exchange, and even regulated transfer of small bioactive molecules, generally water insoluble.

As is well known, albumins may have three different sources, namely, blood serum, milk, or egg white.

Seroalbumin is specific for each species and entails the risk of contamination with other proteins, as well as viral particles, causing problems of transmission of pathologies among species. Furthermore, it is hardly soluble in water and has a high price.

Lactalbumin, present in milk, can only be technically obtained from one source, bovine milk. This makes this protein expensive and difficult to isolate and purify.

Ovalbumin is the main component of egg white, it is inexpensive and easily obtainable. In fact, it constitutes 75 % of hens' egg white, where it is accompanied by minute quantities of avidin, lysozyme, conalbumin, and ovomucoid. Unfortunately, the use of ovalbumin as a pharmaceutical carrier is strongly limited by various factors. Specifically, it is denatured at relatively low temperatures, approximately 56° C, by vigorous stirring, and because avidin, its natural companion, is a toxic factor that deactivates biotin, hence its designation as a harmful factor in egg white, which makes purification very difficult.

To all this we must add another important obstacle for the use of ovalbumin as a pharmaceutical carrier, the allergy and immunotoxicity problems induced, in a significant number of people, by the administration of egg white. Therefore, in order to use ovalbumin in such carriers, it is necessary to conduct treatments which eliminate its intrinsic toxicity, as well as the toxicity associated with its impurities, primarily avidin.

The chemical modification of albumins must also solve the problem of their lack of solubility, without affecting their transport capacity, as well as eliminate the disadvantages resulting from their denaturing by heat, stirring, or the addition of transition metals, many of which are, in fact, biologically active, such as iron, cobalt, etc.

In the attempt to obtain adducts which are stable, chemically defined, and suitable for industrial preparation, the problem of albumins' lack of solubility is not a minor problem. Specifically, ovalbumin entails serious problems of insolubility, and even gelation, that is, the formation of gels, high-viscosity hydrocolloids which do not flow and which are responsible, in industrial production, for the failure of a significant number of batches. As has been mentioned, commercial ovalbumin only contains 75 % of ovalbumin proper by weight, and the accompanying impurities are responsible for the gelling processes in aqueous solution. Specifically, the presence of ovomucoid is what creates the most obstacles for obtaining a perfectly soluble ovalbumin adduct in combination with iron-polyalcohol complexes.

In the field of pharmaceutical research, significant efforts have been devoted to efficiently transporting active therapeutic substances, improving the rate of gastric acceptability, protecting the active molecule from gastric or intestinal degradation, improving therapeutic efficiency, etc.

There are numerous, excellent examples on how to prepare and carry ferric complexes for therapeutic administration in human and veterinary medicine.

Thus, for example, the chelating properties of metals by conalbumin have been described (J. Am. Chem. Soc. 75, 5094, 1953). Some milk derivatives adequately modified in order to solubilise transition metals at physiological pH have also been described (Drug Res. 34 (II) N° 9, 1984), as have trivalent iron complexes with types of carbohydrates (Inorg. Chim. Acta, 124, 55-59, 1986), among them, sorbitol (Inorg. Chim. Acta 80, 251-254, 1983).

Furthermore, the preparation of ferric saccharate and fructate, stabilised and carried by amine complexes, has been described in European patent application EP 0223153 (Mediolanum Farmaceutici), which refers to the best way to therapeutically carry and administer unstable ferric complexes.

Spanish patent application no. 9602180 (15 October, 1996) discloses methods to treat ovalbumin with alkali (NaOH), heating, and controlled oxidation with hydrogen peroxide in solution, the latter with the purpose to deactivate biological risks.

Although it is true that the above-mentioned thermal, alkaline, and oxidation treatment makes it possible to prepare stable adducts with iron polyalcohol complexes, implementation of this method in technical practice demonstrates that the gelling process which appears in a significant number of production batches has not been completely resolved, which represents a major obstacle for the large-scale preparation of these anti-anaemic products. It is true that when commercial ovalbumin is subject to laborious purification processes, such as fractioning with ammonium sulphate (Sorensen et al, Rev. Trav. Lab. Carisberg, 12, 12, (1917)), the problem is resolved, since the ovomucoid is thus eliminated.

But purification by means of ammonium sulphate using Sorensen's method is applicable to egg white as is, but not commercial albumin in simple form, partly because this ovalbumin is denatured by spray drying, and partly because the high ammonium sulphate concentrations used make subsequent dialysis imperative, which is difficult since it involves dialysis of native hydrocolloids.

Consequently, in other words, it is not economically feasible to work with pure ovalbumin; moreover, large-scale purification of commercial ovalbumin is impracticable using the method described to deprive it of ovomucoid. Therefore, significant efforts should be devoted to finding a procedure which, in conjunction with the thermal, alkaline, and oxidation processes, will eliminate *in situ* the undesirable interference provoked by the presence of ovomucoid, without the need for laborious and even unfeasible steps intended to purify the initial ovalbumin. None of the previously cited methods disclose how to correct a natural protein carrier's physiological behaviour by means of chemical treatments which ensure the elimination of undesirable effects, such as allergies, immunotoxicity, viral presence, etc., the generation of industrial preparation methods without the disadvantages of such adducts, and the enhancement of desirable effects such as solubility, gastric protection, regulated iron transfer, etc.

## DESCRIPTION OF THE INVENTION

The method to obtain adducts of soluble ovalbumin with trivalent iron monosaccharide polyalcohol complexes proposed by the invention resolves in a fully satisfactory manner the previously discussed problems, in relation to all the aspects addressed.

According to the invention, a method is provided for the treatment of ovalbumin, which allows for its solubilisation without greatly affecting its molecular weight, as well as the elimination of the allergy and immunotoxicity problems associated, as previously discussed, with said albumin.

Numerous studies and trials have shown that, when albumin is treated under controlled alkalinity conditions, by means of a method which regulates temperatures, times, and the addition of oxidants, a type of soluble albumin is produced which is useful to prepare adducts with trivalent iron polyalcohol compounds.

The method to treat ovalbumin proposed by the invention comprises a set of steps which are described below.

Firstly, a concentrated base solution, preferably sodium hydroxide (NaOH), is added to an albumin suspension in water, in the quantity necessary to obtain a final 1 M NaOH concentration, while stirring vigorously and intensely heating until a temperature between 63° C and 67° C is reached, maintaining said heating and stirring for a period of time between 15 minutes and one hour.

The next step consists of neutralising the previously prepared solution by adding an appropriate acid, for example, HCl, until the pH is adjusted to a value between 8.5 and 10.

Subsequently, an alkaline protease, obtained from fungi or bacteria, is added, in a 1 ‰ proportion over the initial ovalbumin weight, and digesting for two hours, without pH correction, at 48-52°C, subsequently raising the temperature until it reaches 80°C, which causes the destruction of the gelling impurities, to ensure the deactivation of gel-forming capacity and to avoid the degradation of the ovalbumin, primarily serves to hydrolyse the ovomucoid, leaving the ovalbumin practically unaltered.

Finally, $H_2O_2$ is added in a quantity between 4 and 8% of the ovalbumin. The proteolytic treatment performed in the previous step makes it unnecessary to perform any purification process which might be subsequently performed, for instance, ultrafiltration to eliminate salts, peroxide, and low-molecular-weight substances produced by the alkaline heating, such that one may directly proceed to the adduction with ferric complexes, as will be explained in detail further below.

The ovalbumin suspension in water will contain, advantageously, a final weight/volume ovalbumin concentration between 3 % and 7 %. The suspension is prepared by the addition of ovalbumin, normally in powder form, to water in a quantity appropriate to obtain the above-mentioned concentration.

The solution thus produced contains the treated ovalbumin, which is suitable to carry the above-mentioned ferric complexes, for the formation of molecular adducts.

Regarding the operating conditions for the previously described method, it has been demonstrated that:

- An ovalbumin concentration below 3 % (w/v) is not advisable, since its molecular integrity is affected by the alkaline attack, while, at concentrations over 7 %, the high protein concentration leads to solutions with a tendency to form gels, increasingly so as the pH is decreased upon neutralising the NaOH.

- The most appropriate NaOH concentration in the solution is 1M, since it is the appropriate and validated concentration for viral deactivation.

- The heating temperature must be between 63° C and 67° C, since higher temperatures produce alkaline hydrolysis of the protein structure at the above-mentioned NaOH concentration, while, at temperatures below 63° C, alkaline deactivation cannot proceed to completion and, in addition, yields final products with questionable solubility.

- The alkaline treatment time must be between 15 and 60 minutes, since below 15 minutes the resulting products are not adequately soluble, while, above 60 minutes, there is significant alkaline degradation of the protein structure, without preservation of the native protein's molecular weight.

- The alkaline protease must be added at the above-mentioned pH in the mentioned quantity of 1‰ of the ovalbumin weight, since this quantity ensures the destruction of the gelling impurities and inalterability of the ovalbumin itself. In addition, the two-hour digestion period is the appropriate one; a shorter time does not ensure deactivation of the gel-forming capacity; a longer proteolytic treatment initiates ovalbumin degradation as measured by the enzyme.

- $H_2O_2$ must be added in the quantity mentioned, between 4 and 8 % of the ovalbumin, since this quantity is within the limits necessary such that, in compensation for the partial destruction of the $H_2O_2$, in the aqueous medium at pH 8.5 - 10, the biotin deactivating process is equivalent to that of pure water radiolysis. Lower $H_2O_2$ concentrations are not necessarily deactivating, while higher concentrations lead to ovalbumin depolymerisation, significantly modifying its condition as carrier of the above-mentioned ferric complexes. It is worth mentioning that the previously described method for treating albumin does not greatly affect the protein's molecular weight, which makes it possible to operate under thermal conditions wherein the native protein would not be soluble. Treated in this way, the protein is not coagulable by the action of flocculating cations, such as $Fe^{3+}$, thus being capable of carrying ferric complexes in solution which are insoluble in water at the physiological pH. Moreover, the above-mentioned proteolytic enzymatic treatment ensures that ovalbumin will not form gelled products during the industrial preparation processes and during its adduction with the above-mentioned ferric complexes, which would cause considerable economic losses due to the forced discarding of production batches. This treatment makes it possible to eliminate the immunotoxicity and allergy problems caused by the presence of avidin and the protein's allergenic characteristics. The allergic shock problem is eliminated by the alkaline digestion performed at controlled temperature in an aqueous medium. Avidin is destroyed by heating and irradiation, particularly in aqueous solution. In fact, by irradiating water with high-energy electronic x- or δ-rays, several highly reactive radical species are obtained (HO., H., $HO_2$) which, as is well known, denature and fragment the labile protein and nucleic acid molecules. Specifically, biological systems are very sensitive to radiation, particularly when they are irradiated in the presence of oxygen or air. There is a significant technical impossibility for the industrial-scale irradiation of aqueous solutions. The addition of oxidation agents which thermolytically decompose, generating a similar cascade of free radicals, has resolved this technical impossibility to conduct large-scale irradiation of aqueous solutions, thus allowing for the controlled destruction of avidin by means of the peroxidolysis of hydrogen peroxide. Under controlled conditions, this decomposition of radicals does not affect ovalbumin but makes it possible to destroy avidin. The spontaneous thermolytic decomposition of $H_2O_2$ by means of the Haber-Weiss reaction leads to the production of the same radical species as with water radiolysis:

$$O_2 + H_2O_2 \rightarrow OH^- + OH + O_2$$

Furthermore, this careful thermal, alkaline, and oxidation treatment has been validated as the most efficient for the elimination of conventional slow viruses. Using the method to treat ovalbumin provided by this invention, stable adducts of trivalent cation polyalcohol complexes, particularly $Fe^{3+}$, have been produced with the ovalbumin thus treated.

Thus, the method of the invention provides stable adducts of iron (III) polyalcohol complexes with soluble ovalbumin. In a specific embodiment of the method of the invention to produce adducts, said polyalcohol is a polyalcohol derived from a monosaccharide. In a specific preferred embodiment, said polyalcohol is selected from the group formed by manitol and sorbitol.

According to a particular embodiment, the adducts formed according to the method of the invention obey the following general formula:

$$[Fe_x(C_xH_{2x}O_x)]_n \text{ Albumin} \qquad (I)$$

where x is an integer selected among 5, 6, or 12; or else the general formula:

$$[Fe_x(C_xH_{2x+2}O_x)]_n \text{ Albumin} \qquad (II)$$

where x has the previously mentioned meaning, that is, x is selected among 5, 6, or 12.

In a specific preferred embodiment of this invention, the adduct obeys the following molecular formula:

$$110\ [[(FeO)_6\ (C_6H_{14}O_6)]6\ HO]\ 3\ \text{Albumin}$$

Producing said adducts comprises, therefore, a stage wherein the ovalbumin is treated, another stage wherein the trivalent iron polyol complex is formed, and a third stage wherein the adduct is formed by reaction of the treated ovalbumin with said trivalent iron polyalcohol complex.

In order to form the trivalent iron polyalcohol complexes, a source of ferric ions, for example, ferric chloride, is mixed and dissolved in water with the polyalcohol in a basic medium, thus obtaining a solution which is heated to a temperature of approximately 95° C in order to complete complexation; subsequently, it is cooled down.

In order to form the adducts of ovalbumin with iron (III) polyalcohol complexes, the treated ovalbumin solution is added to the ferric complex aqueous solution while stirring.

The high molecular weight resulting adduct is purified by means of an ultrafiltration step which eliminates low molecular weight peptides, low molecular weight ferric complexes, and polysaccharide remains resulting from the catenary structure of the ovomucoid destroyed *in situ* by the above-mentioned proteolytic treatment.

The adduct thus purified is unloaded from the ultrafilter, adjusted to pH 7.8 - 8.2, and lyophilised, without the need to isolate the intermediate products.

The product produced, the adduct of albumin with trivalent iron polyalcohol complexes, may be used for oral administration with therapeutic purposes in iron replacement therapy.

The adducts provided by this invention are appropriate for the treatment of iron deficiency conditions, for example, the treatment of ferropenic anaemia, as well as the contribution or supplementation of iron, even if not due to a pathology, for a person or animal who may need it.

## DESCRIPTION OF THE DRAWINGS

To complement the description being made and in order to help towards a better understanding of the invention's characteristics, in accordance with a preferred embodiment thereof, we attach, as an integral part of said description, a set of drawings wherein the following data have been represented, merely for illustration purposes and without this limiting the scope of the invention:

Figure 1.- Shows a representation of a $^{13}$C-NMR spectrum characteristic of ovalbumin treated in accordance with the procedure disclosed in this invention.

Figure 2.- Shows a $^{13}$C-NMR spectrum characteristic of an adduct of an iron (III) - manitol complex with treated ovalbumin, provided by this invention.

Figure 3.- Shows a permeation chromatogram characteristic of ovalbumin treated in accordance with the procedure disclosed in this invention.

## EXAMPLES OF EMBODIMENTS OF THE INVENTION

The purpose of the following examples is to illustrate specific embodiments of the object of this invention and must not be considered as a limitation on the scope thereof.

Example 1: Adduct between treated ovalbumin and iron (III) - manitol complex.

a) Iron - manitol complex: 31.5 g of $FeCl_3 \cdot 6H_2O$ are dissolved jointly with 7.9 g of manitol in 400 ml of water. A solution of 18 g of NaOH in 48 ml of water is added under vigorous stirring. It is heated to 95° C, letting it spontaneously cool down to ambient temperature.
b) Treated ovalbumin: 37.50 g of commercial ovalbumin in powder form are dispersed under vigorous stirring in 180 ml of water. A solution of 30 g of NaOH in 550 ml of water is heated separately to 65° C. The protein dispersion is added to the alkaline solution and is digested at 65° C for 15 minutes, with the pH being subsequently adjusted to a value between 8.5 - 10 with 6M HCl. While keeping the solution at 50° C and pH 8.5 - 10, Esperase® (NOVO-NORDISK) is added in a quantity of 1 ‰ of the initial albumin (37.5 mg), proteolytically digesting it, under gentle stirring, for 2 hours at 50° C without any pH correction. Once this time has elapsed, the spontaneous pH is 8.5. The solution is heated to 80° C in order to deactivate the remaining enzymatic activity, the pH is adjusted to 10 with 10M NaOH and, when the temperature goes down to 65° C, 2 ml/l of 30 % w/v $H_2O_2$ are added. It is allowed to cool down to ambient temperature, which precipitates an easily filterable white floccule, leaving a limpid, amber-colour, odourless solution.

c) Ovalbumin - iron - manitol adduct: the aqueous iron manitol solution is added to the protein solution produced in b), under gentle stirring. It is adjusted to pH 10 with 2M HCl and filtered with Supercel as a filtering aid, which produces a clear, dark red solution. It is concentrated by means of ultrafiltration (Cut-Off 10000 Da)) to one-third of the initial volume, and diafiltered at constant volume against 10 volumes of distilled water. It is unloaded from the ultrafiltration equipment, adjusted to pH 8.0 - 8.2 with 2M HCl, filtered by means of a depth plate, and lyophilised. 20 g of a lyophilised powder are produced, which is dark red in colour and exhibits the following characteristic analysis:

| | |
|---|---|
| Loss by drying: | 2 % |
| Total iron: | 15.3 % |
| Organic iron: | 15.2 % |
| Total N: | 8.5 % |
| Ammonium N: | 800 ppm |
| 1 % solubility: | limpid |
| pH at 1 %: | 8.0 |

Molecular weight (HPSEC): 200,000 Da[*]
(*)Bio Sep Sec 4000 column (300 x7.5); solvent 0.1M NaCl, 0.5M $PO_4H_2Na$, pH 7.4; detection 280 nm. Calibration conducted by means of known molecular weight standards: 13000 Da Cyochrome, 65000 Da Bovine seroalbumin, 230000 Da Catalase, 440000 Da Equine ferritin.

Example 2: Adduct of treated ovalbumin with iron (III) - sorbitol complex.

a) Iron (III) - sorbitol complex: 31.50 g of Fe $Cl_36H_2O$ are dissolved in 400 ml of distilled water jointly with 7.90 g of sorbitol. A 10M NaOH solution (45 ml) is added under vigorous stirring. It is heated, under stirring, to 95° C, letting it spontaneously cool down to ambient temperature.

b) Treated ovalbumin: 37.50 g of commercial ovalbumin in powder form are treated under identical conditions as in example 1.

c) Adduct treated ovalbumin - iron (III) - sorbitol: the solution produced in a) is homogenised under stirring with the protein solution produced in b), subsequently purifying it by ultrafiltration according to example 1. The purified solution thus produced is lyophilised, and 20.6 g of a soft powder are produced, dark red in colour, which exhibits the following characteristic analysis:

| | |
|---|---|
| Loss by drying: | 2 % |
| Total iron: | 15.6 % |
| Organic iron: | 15.2 % |
| Total N: | 8.6 % |
| pH at 1 %: | 8.0 |

Subsequently, the chemical and spectroscopic characterisation of the intermediate and final products produced is performed.

Regarding the treated ovalbumin, using the production method explained in example 1, the intermediate "treated ovalbumin" is not isolated nor does it require any purification before adduction with the ferric complex solution, and this is due to the advantages involved in the controlled enzymatic proteolysis of ovalbumin.

In order to obtain the intermediate product in question, the starting point is commercial ovalbumin, which is subject to the alkaline digestion, proteolytic, and oxidation treatments, and the material thus obtained is subsequently purified in order to characterise it. As can be seen in examples 1 and 2, this purification is not necessary for preparation purposes, hence the simplicity of the production method described in the invention.

In order to isolate it and analytically characterise it, the following protocol is used: 37.50 g of commercial ovalbumin in powder form are dispersed, under vigorous stirring, in 180 ml of distilled water and poured, while vigorously stirring, on a solution composed of 30 g of NaOH in 550 ml of water at 65° C. It is digested, under stirring, at 65° C for 15 minutes, the pH is adjusted to 10 with 6M HCl, and it is cooled down to 48 - 52° C. 37 mg of Eperase® are added, digesting for two hours at this temperature, without pH correction, allowing the latter to freely evolve. After 2 hours have elapsed, it is heated to 80° C in order to deactivate all the enzymatic remains, it is cooled down to 65° C, and 2 ml/l of 30 % $H_2O_2$ are added. It is cooled down to ambient temperature, filtered in order to obtain a limpid, bright solution, which cannot occur without enzymatic proteolysis, and subsequently diafiltered in an ultrafilter until there is a negative reaction to chlorides and the biuret reagent in the permeates; this is achieved by means of 15 volumes of distilled water. It must be mentioned that, in the course of the enzymatic hydrolysis, the accompanying ovomucoid, which is responsible for the undesirable gelling processes in industrial batches during technical preparation of the above-mentioned adducts, is destroyed *in situ*. This is verified because, if a Molisch reaction is performed on drops of the 10000 Da permeate during the ultrafiltration process mentioned herein, a strong positive carbohydrate reaction is produced, most likely due to the products of the proteolytic degradation of the ovomucoid glycoprotein, which is destroyed by the controlled enzymatic attack. If the enzymatic attack is not conducted, the above-mentioned permeates produce a negative Molisch reaction. Similarly, the permeates' reaction is negative if the enzymatic attack described herein is performed on ovalbumin purified with ammonium sulphate using Sorensen's method.

The presence of oligosaccharides in the 10 kDa permeates is not due to the enzymatic digestion of ovalbumin, nor the alkaline digestion of commercial ovalbumin, but to the proteolytic degradation of the impurities which, in technical practice, are responsible for gelling processes, coagulation in the reaction with iron, etc.

The lyophilised product thus produced that is, the treated ovalbumin intermediate, exhibits the following characteristic analysis:

|  | **Treated ovalbumin** | **Ovalbumin treated without proteases** | **Ovalbumin literature data** |
|---|---|---|---|
| **Total N** | 14.0 % | 14.2 % | 14.0% |
| **Total Na** | 1.9 % | 2.3 % |  |
| **C** | 48.0 % | 47.96 % | 46.2 % |
| **H** | 7.2% | 7.35% | 7.7% |
| **O** | 28.9% | 28.2% |  |

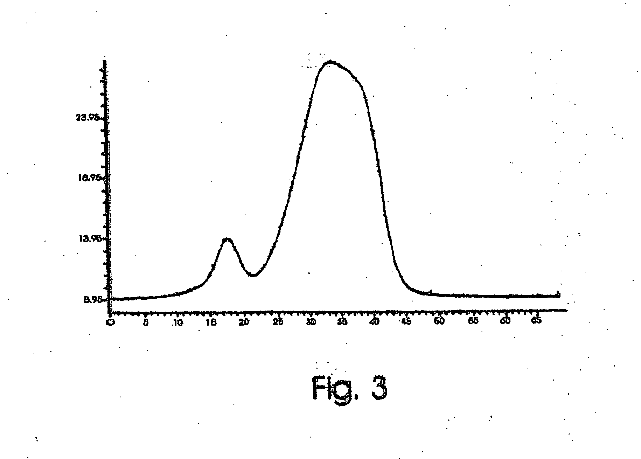

The molecular weight, obtained by permeation chromatography (HPSEC), is 43000 Da in the case of a product treated without proteases, 43000 Da in the case of a product subject to proteolytic treatment, as in this invention, and 45000 Da for native ovalbumin. The HPSEC operating conditions are the ones mentioned in example 1. The characteristic permeation chromatograms are shown in figure 3.

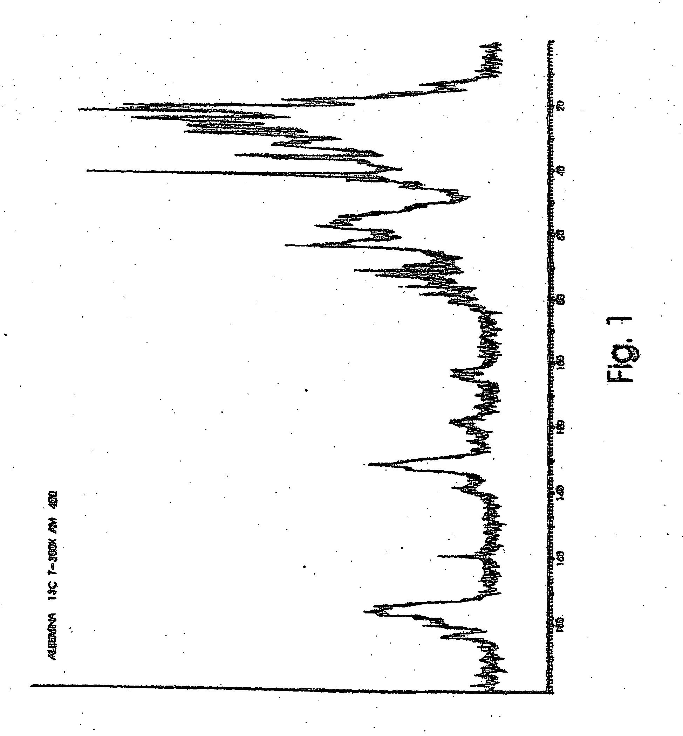

The $^{13}$C-NMR spectrum characteristic of this intermediate is represented in figure 1, and shows the typical complexity of protein macromolecular structures. The spectrum may be divided into three regions:

- aliphatic signals (10 - 80 ppm)
- aromatic signals (120 - 140 ppm)
- carboxylic and amidic signals (150 - 190 ppm) minor signals which recognise remanent carbohydrate structures are visible between 70 - 110 ppm.

Regarding the adduct of treated ovalbumin with iron (III) - manitol complex, produced according to the procedure described in example 1, it exhibits the following characteristic analysis:

a) Elemental analysis: 33.50 % C, 5.0 % H, 8.6 % N, 1.2 % Cl, 18.3 % Fe, 1.83 % Na, and 32.57 % O.

b) Molecular weight: following the previously described HPSEC method, the value found is 210000 Da.

c) Ovalbumin / iron (III) - manitol complex relation: the percentage of N found in the adduct (8.62 %), and the C/N relation in the treated ovalbumin (3.42), allow us to conclude that the percentage corresponding to the protein fraction in the adduct is:

$$8.62 \times 3.42 = 29.48 \% \text{ C belonging to the protein.}$$

The remaining % C corresponds to manitol's contribution, i.e.: 33.53 - 29.48 = 4.05 % C. Furthermore, if the % C in the treated ovalbumin is 47.96 %, the protein total will be:

$$\frac{29.48 x 100}{47.96} = 61.5 \% \text{ treated ovalbumin}$$

and for manitol:

$$\frac{4.05 x 182}{6 x 12} = 10.2 \% \text{ manitol}$$

therefore, the adduct contains 61.5 % treated ovalbumin and 28.5 % iron (III) - manitol complex.

d) Molecular formula:

$$110\{[(FeO)_6(C_6H_{14}O_6)]6HO\}3 \text{ protein}$$

and, in general, adducts with the generic formula (I):

$$[Fe_x(C_xH_{2x}O_x)]_n \text{ Albumin} \qquad (I)$$

or else, with the generic formula (II):

$$[Fe_x(C_xH_{2x+2}O_x)]_n \text{ Albumin} \qquad (II)$$

where x is 5, 6, or 12.

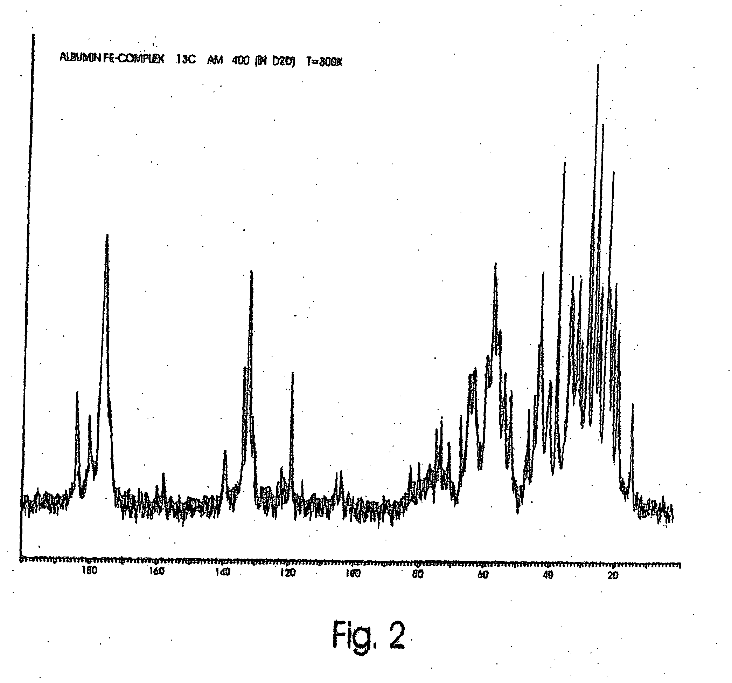

e) $^{13}$C-NMR: the corresponding spectrogram, represented in figure 2, shows peculiar modifications in the protein's spectrum profile, which demonstrates that there are interactions between ovalbumin and the iron (III) - manitol complex, as well as structural modifications in the protein unit.

**Claims**

1. Procedure to obtain adducts of soluble ovalbumin with trivalent iron polyalcohol complexes which have the following general

$$[Fe_x(C_xH_{2x}O_x)]_n \text{ Albumin} \qquad (I)$$

or else the general formula:

$$[Fe_x(C_xH_{2x+2}O_x)]_n \text{ Albumin} \qquad (II)$$

where x is an integer selected among 5, 6, or 12,
comprising the steps of treating ovoalbumin, forming the trivalent iron polyol complex and obtaining said adducts by reaction of the treated ovalbumin with a trivalent iron polyalcohol complex **characterised in that** the treatment of the ovalbumin comprises the steps of:

a) adding a solution of a concentrated base, preferably sodium hydroxide (NaOH), to an albumin suspension in water, in the quantity necessary to obtain a final 1 M NaOH concentration, while stirring vigorously and intensely heating until a temperature between 63°C and 67°C is reached, keeping said heating and stirring for a period of time between 15 minutes and one hour;

b) neutralising the previously prepared solution by adding an appropriate acid, for example, HCl, until the pH is adjusted to a value between 8.5 and 10;

c) adding an alkaline protease obtained from fungui or bacteria in a 1 ‰ proportion over the initial ovalbumin weight and digesting for two hours, without pH correction, at 48 - 52°C, subsequently raising the temperature until it reaches 80°C, which causes the destruction of the gelling impurities, to ensure the deactivation of the gel-forming capacity and to avoid the degradation of the ovalbumin.

d) subsequently adding $H_2O_2$ in a quantity between 4 and 8% of the ovalbumin.

2. Procedure according to claim 1 **characterised in that** the ovalbumin suspension in water contains a final ovalbumin concentration between 3% and 7% w/v.

3. Procedure according to claim 1 **characterized in that** the polyalcohol compound used to obtain the trivalent iron polyalcohol complexes is a polyalcohol derived from monosaccharide carbohydrates.

4. Procedure according to claim 1 **characterised in that**, in order to form the trivalent iron polyalcohol complexes, a source of ferric ions and the polyalcohol in a basic medium are mixed and dissolved in water, thus producing a solution that is heated to a temperature of approximately 95°C in order to complete complexation, subsequently allowing it to cool down.

5. Procedure according to claim 1 **characterised in that** to form the adducts of ovalbumin with iron (III) complexes, the solution of treated ovalbumin is added to the ferric complex aqueous solution under stirring.

6. Procedure according to claim 1 **characterised in that** includes an additional step to purify the resulting adduct by means of concentration by ultrafiltration followed by diafiltering with distilled water, in an ultrafilter with a 10000 Da molecular weight ultrafiltration membrane.


**Patentansprüche**

1. Verfahren zum Gewinn von Addukten von lösbarem Eieralbumin mit trivalenten Eisen-Polyalkohol-Komplexen folgender allgemeiner Formel:

$$[Fe_x(C_xH_{2x}O_x)]_n \text{ Albumin} \qquad (I)$$

oder auch der allgemeinen Formel:

$$[Fe_x(C_xH_{2x+2}O_x)]_n \text{ Albumin} \qquad (II)$$

in der x eine Ganzzahl von 5, 6 oder 12 ist,
Verfahren, das folgende Schritte umfasst: Verarbeitung des Eieralbumins unter Bildung des trivalenten Eisen-Polyol-Komplexes und Erhalt von besagten Addukten durch die Reaktion des behandelten Eieralbumins mit einem trivalenten Eisen-Polyalkohol-Komplex, **dadurch gekennzeichnet dass** die Verarbeitung des Ovalbumin folgende Schritte umfasst:

a) Hinzugabe einer Lösung einer konzentrierten Base, bevorzugt Natriumhydroxyd (NaOH), zu einer Albuminsuspension in Wasser in der notwendigen Menge, um eine Endkonzentración von 1M NaOH zu erhalten unter kräftigem Rühren und mit einer starken Erhitzung bis zu einer Temperatur zwischen 63°C und 67°C; diese Erhitzung und das Rühren muss über einen Zeitraum von 15 Minuten bis zu einer Stunde durchgeführt werden;

b) Neutralisation der vorher erhaltenen Lösung durch Hinzufügen einer passenden Säure, z. B. HCl, bis der pH-Wert auf einen Wert von 8,5 bis 10 eingestellt ist;

c) Hinzufügen einer alkalischen Pilz- oder Bakterienprotease in einer Proportion von 1 ‰ mit bezug auf das anfängliche Gewicht des Eieralbumins und zweistündiges Digerieren ohne Einstellung des pHs, bei 48 bis 52°C, danach Temperaturerhöhung bis zu 80°C, wodurch die Gelierungsunreinheiten zerstört werden, um die Desaktivierung der Gelierungsfähigkeit zu sichern und die Zersetzung des Eieralbumin zu vermeiden.

d) Danach Hinzufügen von $H_2O_2$ in einer Menge von 4 bis 8% des Eieralbumins.

2. Verfahren gemäss Anspruch 1, **dadurch gekennzeichnet, dass** die Suspension von Eieralbumin in Wasser eine Endkonzentration an Eieralbumin von 3% bis 7% Volumengewicht enthält.

3. Verfahren gemäss Anspruch 1, **dadurch gekennzeichnet, dass** die verwendete Polyalkoholverbindung zum Erhalt des trivalenten Eisen-Polyalkohol-Komplexes ein Polyalkohol ausgehend von Monosaccharide-Kohlenhydraten ist.

4. Verfahren gemäss Anspruch 1, **dadurch gekennzeichnet, dass** zur Bildung des trivalenten Eisen-Poly-Alkohol-Komplexes eine Ferri-Ionen-Quelle und der Polyalkohol in einem basischem Mittel gemischt und in Wasser gelöst werden, um so eine Lösung zu erhalten, die bis auf eine Temperatur von ca. 95 °C erhitzt wird, um die Komplexblidung zu vervollständigen, wonach man sie abkühlen lässt.

5. Verfahren gemäss Anspruch 1, **dadurch gekennzeichnet, dass** zur Bildung der Eieralbuminaddukte mit Eisen (III)-Komplexen die Lösung von aufbereitetem Eieralbumin unter Rühren zu einer wässrigen Lösung des Ferri-Komplexes hinzugefügt wird.

6. Verfahren gemäss Anspruch 1, **dadurch gekennzeichnet dass,** ein zusätzlicher Schritt vorgesehen ist, um das gewonnene Addukt zu reinigen, und zwar mittels Konzentration durch Ultrafiltration gefolgt von einer Diafiltrierung mit destilliertem Wasser in einem Ultrafilter mit einer Membrane für Ultrafiltration von 10000Da Molekulargewicht.

**Revendications**

1. Procédure pour obtenir des adduits d'ovalbumine soluble avec des complexes fer-polyalcool trivalents ayant la formula générale suivante

$$[Fe_x(C_xH_{2x}O_x)]_n \text{ Albumine} \qquad (I)$$

ou la formule générale

$$[Fe_x(C_xH_{2x}+_2O_x)]_n \text{ Albumine} \qquad (II)$$

dans laquelle x est un entier choisi parmi 5, 6 ou 12, comprenant les étapes de traitement de l'ovoalbumine, la formation du complexe fer-polyol trivalent et l'obtention desdits adduits au moyen de la réaction de l'ovalbumine traitée avec un complexe fer-polyalcool trivalent **caractérisée en ce que** le traitement de l'ovalbumine comprend les étapes de :

a) ajouter une solution d'une base concentrée, de préférence hydroxyde de sodium (NaOH) à une suspension d'albumine dans de l'eau, en une quantité nécessaire pour obtenir une concentration finale de 1M de NaOH, tout en l'agitant vigoureusement et en la chauffant intensément jusqu'à ce qu'une température située entre 63° C et 67° C soit atteinte, maintenant ledit chauffage et ladite agitation durant une période de temps de 15 minutes à une heure ;

b) neutraliser la solution préalablement préparée moyennant l'adition d'un acide approprié, par exemple HCl, jusqu'à ce que le pH soit ajusté à une valeur située entre 8.5 et 10 ;

c) ajouter une protéase alcaline obtenue à partir de champignons ou de bactéries suivant une proportion de 1 ‰ par rapport au poids initial d'ovalbumine et sa digestion pendant deux heures, sans correction du pH, à 48-52° C, en augmentant postérieurement la température jusqu'à ce qu'elle atteigne 80° C, ce qui provoque la destruction des impuretés de gélification, pour assurer la désactivation de la capacité de formation de gel et éviter la dégradation de l'ovalbumine.

d) ajouter postérieurement $H_2O_2$ en une quantité située entre 4 et 8 % de l'ovalbumine.

2. Procédure selon la revendication 1 **caractérisée en ce que** la suspension d'ovalbumine dans l'eau contient une concentration finale d'ovalbumine située entre 3 % et 7 % poids/volume.

3. Procédure selon la revendication 1 **caractérisée en ce que** le composé de polyalcool utilisé pour obtenir les complexes fer-polyalcool trivalents est un polyalcool provenant de carbohydrates sous la forme de monosaccharides.

4. Procédure selon la revendication 1 **caractérisée en ce que,** afin de former les complexes fer-polyalcool trivalents, une source d'ions ferreux et le polyalcool sont mélangés dans un milieu basique et dissous dans de l'eau, produisant ainsi une solution qui est chauffée à une température d'environ 95° C afin de réaliser la complexation, la laissant postérieurement se refroidir.

5. Procédure selon la revendication 1 **caractérisée en ce que** pour former les adduits d'ovalbumine avec les complexes du fer (III), la solution d'ovalbumine traitée est ajoutée sous agitation à la solution aqueuse du complexe ferreux.

6. Procédure selon la revendication 1 **caractérisée en ce qu'**elle inclut une étape additionnelle pour purifier l'adduit résultant au moyen de la concentration par ultrafiltration suivie de la diafiltration avec de l'eau distillée, dans un ultrafiltre avec une membrane d'ultrafiltration d'un poids moléculaire de 10.000 Da.

Fig. 1

EP 1 655 308 B1

ALBUMIN FE-COMPLEX   13C   AM   400  (IN D2O)   T=300K

Fig. 2

Fig. 3

**EP 1 655 308 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0223153 A
- ES 9602180

### Non-patent literature cited in the description

- *J. Am. Chem. Soc.,* 1953, vol. 75, 5094
- *Drug Res.,* 1984, vol. 34 (9
- *Inorg. Chim. Acta,* 1986, vol. 124, 55-59
- *Inorg. Chim. Acta,* 1983, vol. 80, 251-254
- **Sorensen et al.** *Rev. Trav. Lab. Carisberg,* 1917, vol. 12, 12